Europäisches Patentamt

European Patent Office

Office européen des brevets

(11) Veröffentlichungsnummer: **0 077 449**
**B1**

(12) # EUROPÄISCHE PATENTSCHRIFT

(45) Veröffentlichungstag der Patentschrift:
**29.05.85**

(51) Int. Cl.⁴: **G 01 N 33/68**, C 12 Q 1/60

(21) Anmeldenummer: **82107718.7**

(22) Anmeldetag: **23.08.82**

(54) **Verfahren zur Bestimmung der beta-Lipoproteinfraktion (LDL) in Körperflüssigkeiten.**

(30) Priorität: **27.08.81 DE 3133937**

(43) Veröffentlichungstag der Anmeldung:
**27.04.83 Patentblatt 83/17**

(45) Bekanntmachung des Hinweises auf die Patenterteilung:
**29.05.85 Patentblatt 85/22**

(84) Benannte Vertragsstaaten:
**AT BE CH DE FR GB IT LI LU NL SE**

(56) Entgegenhaltungen:
**DE - A - 1 956 848**
**DE - A - 3 007 764**
**DE - A - 3 010 400**
**US - A - 4 110 077**
**US - A - 4 234 317**

(73) Patentinhaber: **BOEHRINGER MANNHEIM GMBH,**
**Patentabteilung, Abt. E Sandhofer**
**Strasse 112-132 Postfach 31 01 20,**
**D-6800 Mannheim 31 Waldhof (DE)**

(72) Erfinder: **Maier, Josef, Schmädlstrasse 15,**
**D-8120 Weilheim (DE)**
Erfinder: **Gloger, Manfred, Dr., Karwendelstrasse 6,**
**D-8120 Weilheim (DE)**
Erfinder: **Dräger, Brigitte, Dr., Sprungleitenweg 2,**
**D-8132 Tutzing (DE)**

(74) Vertreter: **Weickmann, Heinrich, Dipl.-Ing. et al,**
**Patentanwälte Dipl.-Ing. H.Weickmann Dipl.-Phys.Dr.**
**K.Fincke Dipl.-Ing. F.A.Weickmann Dipl.-Chem. B. Huber**
**Dr.-Ing. H. Liska Dipl.-Phys.Dr. J. Prechtel**
**Postfach 860820, D-8000 München 86 (DE)**

**Beschreibung**

Die Erfindung betrifft ein Verfahren und ein Reagens zur Bestimmung der $\beta$-Lipoproteinfraktion (Low Density Lipoprotein (LDL)) in Körperflüssigkeiten.

Hypercholesterinämie und Hypertriglyceridämie begünstigen die Entstehung der Atherosklerose und des Herzinfarkts. Die Bestimmung von Cholesterin und Triglyceriden im Serum gehören daher zu den am häufigsten durchgeführten Tests im klinisch-chemischen Routinelabor.

Zahlreiche Untersuchungen zum Feststoffwechsel kommen zu dem Schluß, daß das individuelle Coronarrisiko besser erfaßt werden kann, wenn nicht nur die Veränderung im Triglycerid- und Cholesterinspiegel bestimmt, sondern die zugrundeliegenden pathologischen Verschiebungen im Lipoproteinmuster ermittelt werden (Münch. med. Wschr. 121 (1979) 1639).

Die bekannten Plasmalipoproteine enthalten einen unterschiedlich hohen Anteil an Protein, Phospholipiden, Cholesterin und Triglyceriden. Sie lassen sich aufgrund ihres Verhaltens (unterschiedliche Dichte) in der analytischen Ultrazentrifuge in drei verschiedene Klassen unterteilen:

Prä-$\beta$-Lipoprotein   = VLDL (Very Low Density Lipoprotein)
$\beta$-Lipoprotein   = LDL (Low Density Lipoprotein)
$\alpha$-Lipoprotein   = HDL (High Density Lipoprotein)

Die Untersuchung der Funktion der Lipoproteine ergab, daß LDL innerhalb der Lipoproteine die entscheidende atherogene Komponente darstellt, bei deren Erhöhung im Blut ein gesteigertes Risiko für eine coronare Krankheit vorliegt. Die frühzeitige Erkennung und Bekämpfung dieses Zustands ist von großer Bedeutung. Es besteht daher ein Bedarf nach einem praktikablen Verfahren zur quantitativen Bestimmung der LDL-Konzentration im Serum und Plasma.

Bisher wurden für die Bestimmung der LDL-Lipoproteinfraktion im wesentlichen vier Methoden eingesetzt, die jedoch Nachteile besitzen:

## 1. Ultrazentrifugation

Dieses Verfahren ist für ein Routinelabor nicht geeignet, da man hierzu eine spezielle Geräteausrüstung benötigt und die Durchführung eine äußerst sorgfältige Arbeitstechnik und einen sehr hohen Zeitaufwand erfordert. Somit blieb dieses Analysenverfahren bisher nur dem Labor der forschenden Medizin vorbehalten.

## 2. Fällungsreaktion

Der LDL-Gehalt kann durch franktionierte Fällung mit einem Polyanion, wie Heparin-Na oder Dextransulfat und einem zweiwertigen Kation, wie $Ca^{++}$, $Mn^{++}$, $MG^{++}$, ebenfalls bestimmt werden. Die Lipoproteine lassen sich nämlich mit steigender Konzentration des Polyanions in der folgenden Reihenfolge ausfällen: VLDL, LDL und HDL. Dieses Verfahren erfordert jedoch zwei Arbeitsschritte und ist somit unpraktikabel und nicht automatisierbar: VLDL wird in einem ersten Fällungsschritt abgetrennt, anschließend wird durch Erhöhung der Konzentration des Fällungsmittels die LDL-Lipoproteinfraktion gefällt und turbidimetrisch bestimmt (H. Okabe, X. Int. Congr. of Clin. Chem., Mexico (1978)).

## 3. Bestimmung der LDL-Konzentration über die Friedewald-Formel

Bei diesem Verfahren bestimmt man den Triglycerid-, Cholesterin- und HDL-Cholesteringehalt der Probe und berechnet daraus den Gehalt an LDL-Cholesterin nach dem Verfahren von Friedewald, Clin. Chem. 18 (1972) 499. Dieses umständliche Verfahren liefert keine exakten Werte, insbesondere bei triglyceridreichen Seeren.

## 4. Qualifizierung nach elektrophoretischer Trennung und Polyanionenfällung

Dieses Verfahren jedoch ist zeitaufwendig und erfordert eine eigene Elektrophorese-Apparatur sowie ein Densitometer zur Auswertung (Lab. Med. 1 (1977) 145).

Der Erfindung lag daher die Aufgabe zugrunde, ein praktikables, automatisierbares Verfahren zu schaffen, mit dem LDL im Routinelabor unmittelbar bestimmt werden kann. Gelöst wird diese Aufgabe erfindungsgemäß durch ein Verfahren zur Bestimmung der $\beta$-Lipoproteinfraktion (LDL) in Körperflüssigkeiten, wie Serum oder Plasma, welches dadurch gekennzeichnet ist, daß man die LDL-Fraktion aus der Körperflüssigkeit durch Zusatz eines Polyvinylsulfates mit einwertigem Kation selektiv fällt und bestimmt.

Das erfindungsgemäße Verfahren beruht auf der Auffindung der überraschenden Tatsache, daß LDL in Gegenwart von VLDL und HDL selektiv und praktisch quantitativ durch Zusatz eines Polyvinylsulfates mit einwertigem Kation gefällt und bestimmt werden kann. Dies ist überraschend, da nach den bisher publizierten Untersuchungen bei Ausfällung von LDL stets VLDL mitfällt, wenn es nicht vorher abgetrennt wurde (M. Burgstein, H. R. Scholnick in »Protides of the Biological Fluids«, Ed. Peeters, S. 21—28 (1972); Ärztl. Lab. 23, 101—110 (1977)).

Vorzugsweise erfolgt die erfindungsgemäße Fällungsbehandlung direkt im nicht vorbehandelten Vollserum. Der Niederschlag wird abgetrennt, zweckmäßig durch Zentrifugieren.

Unter Polyvinylsulfat wird im Rahmen der Erfindung ein sich vom Polyvinylalkohol ableitendes Polymeres verstanden, bei dem die Hydroxylgruppen mit Schwefelsäure verestert vorliegen. Die Molekülgröße ist ohne merklichen Einfluß auf das Verfahren, solange das Produkt noch ausreichend wasserlöslich ist. Zweckmäßig sollten jedoch mindestens 70% der zugrunde liegenden Vinylalkoholeinheiten eine Sulfatestergruppe aufweisen. Vorzugsweise soll der Veresterungsgrad 80% oder mehr, besonders bevorzugt 90% oder mehr ausmachen.

Die jeweils erforderliche Polyvinylsulfatmenge läßt sich durch einfache Vorversuche leicht bestimmen. Bevorzugt werden 0,1 bis 0,01% Gewicht pro Volumen Probelösung an Polyvinylsulfat zugesetzt. Als zweckmäßig erwies sich der Zusatz des Polyvinylsulfates in Form einer verdünnten wäßrigen Lösung mit etwa 1 bis 20 Gramm Polyvinylsulfat (PVS) pro Liter. Die Erfindung läßt sich aber auch mit konzentrierteren Lösungen und auch mit Zugabe von PVS in fester Form durchführen, wenn die Probelösung ausreichend gerührt werden kann. Da in der Regel jedoch die Probelösungsmengen sehr gering sind, wird der Zusatz in Form einer verdünnten Lösung bevorzugt. Beispielsweise werden 200 Volumenteile Serum mit 40 Volumenteilen der 0,1 bis 1%igen PVS-Lösung versetzt. Ganz besonders gute Ergebnisse wurden mit einer 0,2 bis 0,5%igen wäßrigen Lösung, welche im Volumenverhältnis 1 : 5 der Probelösung zugesetzt wird, erhalten.

Bevorzugt setzt man der Probelösung außer dem PVS auch einen Polyglykolmethyläther oder Polyvinylpyrrolidon zu. Bei Anwendung dieser Kombination ist die Fällung besonders gut reproduzierbar. Der Polyglykolmethyläther oder/und das Polyvinylpyrrolidon werden der Probelösung vorzugsweise in einer Menge von 5 bis 10 Vol-% zugesetzt.

Beim Verfahren der Erfindung ist es wesentlich, daß keine zweiwertigen Kationen in meßbaren Mengen vorhanden sind. Bei normalerweise im Serum vorhandenen Mengen an zweiwertigen Kationen, bei denen es sich um geringe Spuren handelt, treten jedoch noch keine Störungen auf, so daß dieses Erfordernis vernachlässigt werden kann. Da jedoch das Auftreten anormaler Proben mit einem ungewöhnlich hohen Gehalt an zweiwertigen Kationen nicht ausgeschlossen werden kann, wird vorzugsweise außer den genannten Substanzen auch noch ein Komplexbildner für mehrwertige Kationen zugesetzt. Bevorzugt werden hierfür Polyaminacetate wie z. B. Äthylendiaminotetraessigsäure (EDTA). Ein derartiger Komplexbildner wird bevorzugt in solcher Menge eingesetzt, daß seine Konzentration in der Probelösung 0,01 bis 0,001 Mol/l beträgt.

Die Auswertung der erfindungsgemäß gefällten LDL-Fraktion erfolgt zweckmäßig, indem man eine der bekannten Methoden zur Bestimmung von Lipoproteinen anwendet. Vorzugsweise wird das im Lipoprotein enthaltene Cholesterin nach hierfür bekannten Methoden bestimmt, beispielsweise durch Oxidation mit Cholesterinoxidase und Messung des bei der Oxidation gebildeten $H_2O_2$. Die Durchführung dieser Bestimmung erfolgt zweckmäßig entweder so, daß man das Gesamtcholesterin in der Probe sowie das Cholesterin im Fällungsüberstand bestimmt und aus der Differenz die LDL-Menge entnimmt oder den LDL-Niederschlag auflöst und in der so erhaltenen Lösung die Cholesterinbestimmung durchführt. Die gemessene Cholesterinmenge ist der Lipoproteinmenge analog, da die Cholesterinmenge in den Lipoproteinfraktionen konstant ist.

Das PVS wird vorzugsweise als Kaliumsalz eingesetzt, die anderen einwertigen Kationen insbesondere Natrium, Lithium und Ammonium sind jedoch in gleicher Weise verwendbar.

Ein weiterer Gegenstand der Erfindung ist ein Reagenz zur Bestimmung der $\beta$-Lipoproteinfraktion in Körperflüssigkeiten, welches dadurch gekennzeichnet ist, daß es Polyvinylsulfat enthält. Vorzugsweise enthält es zusätzlich einen Polyvinylmethyläther oder/und Polyvinylpyrolidon. Gemäß einer weiteren bevorzugten Ausführungsform enthält das Reagenz außerdem einen Komplexbildner für mehrwertige Kationen, besonders bevorzugt ein Polyaminacetat.

Ein typisches Reagenz der oben beschriebenen bevorzugten Art enthält 0,1 bis 0,01 Gew.-% Polyvinylsulfat, 5 bis 10 Vol.-% Polyglycolmethyläther oder/und Polyvinylpyrolidon sowie Komplexbildner für eine Konzentration von 0,01 bis 0,001 Mol, jeweils auf das Serumvolumen bezogen.

Das erfindungsgemäße Verfahren und Reagenz zeichnen sich durch besondere Einfachheit und Zuverlässigkeit aus. Der Vergleich mit anerkannten, wesentlich umständlicheren Bestimmungsmethoden ergab eine ausgezeichnete Übereinstimmung der Resultate. Das folgende Beispiel erläutert die Erfindung:

**0 077 449**

Beispiel

A)  Herstellung des Fällungsreagenzes

Lösung I, PVS/Polyäthylenglycolmethyläther

| 34 ml | PVS-Lösung (c = 3 g/l) |
| 16 ml | Polyäthylenglycolmethyläther (MG 190—550) |
| 50 ml | EDTA (0,01 M) |

Lösung II, PVS/Plasdone⊗

| 34 ml | PVS-Lösung (c = 3 g/l) |
| 16 ml | H₂O |
| 50 ml | EDTA (0,01 M) |
| 6,5 g | Plasdone®, K 29—32 (Polyvinylpyrrolidon) |

B)  Präzipitation

| In Zentrifugengläser pipettieren | |
| --- | --- |
| Probe | 200 µl |
| Fällungsreagenz I oder II | 100 µl |

mischen, 15 Minuten bei Raumtemperatur stehen lassen und 2 min bei 10 000 g (oder 15 min bei 1500 g) zentrifugieren. Nach Zentrifugation wird der Überstand, der je nach VLDL- und Chylomikronen-Gehalt ein klares oder trübes Aussehen hat, abgetrennt und für die Cholesterinbestimmung mit der CHOD-PAP-Methode eingesetzt. Die CHOD-PAP-Methode beruht auf der Oxidation von Cholesterin mit Cholesterinoxidase und photometrischer Bestimmung von dabei gebildetem H₂O₂ durch Farbreaktion mit Phenol und 4-Aminoantipyrin.

C)  Cholesterinbestimmung

| Wellenlänge: | Hg 546 nm (470—560 nm) |
| Spektralphotometer: | 500 nm |
| Küvette: | 1 cm Schichtdicke |
| Inkubationstemperatur: | 20—25° C oder 37° C |

Messung gegen Reagenzien-Leerwert (RL)
Für jede Meßreihe genügt ein Reagenzien-Leerwert.

| In Reagenzgläser pipettieren | RL | Probe |
| --- | --- | --- |
| Überstand | — | 50 µl |
| CHOD-PAP-Reagenz | 2000 µl | 2000 µl |

mischen, RL und Probe 20 min bei 20 bis 25° C oder 12 min bei 37° C inkubieren. Innerhalb einer Stunde Extinktion der Probe gegen RL messen (E_Probe).

**Patentansprüche**

1. Verfahren zur Bestimmung der β-Lipoproteinfraktion (LDL) in Körperflüssigkeiten, insbesondere im Serum, dadurch gekennzeichnet, daß man die LDL-Fraktion aus der Probe durch Zusatz eines Polyvinylsulfates mit einwertigem Kation selektiv fällt und LDL in üblicher Weise bestimmt.

4

2. Verfahren nach Anspruch 1, dadurch gekennzeichnet, daß man der Serumprobe zusätzlich einen Polyglykolmethyläther oder Polyvinylpyrrolidon zusetzt.

3. Verfahren nach Anspruch 1 oder 2, dadurch gekennzeichnet, daß man ein wasserlösliches Polyvinylsulfat verwendet, in welchem mindestens 70% der zugrunde liegenden Vinylalkoholeinheiten eine Sulfatgruppe tragen.

4. Verfahren nach einem der vorhergehenden Ansprüche, dadurch gekennzeichnet, daß man der Serumprobe 0,1 bis 0,01% Gew./Vol. Polyvinylsulfat zusetzt.

5. Verfahren nach einem der Ansprüche 2 bis 4, dadurch gekennzeichnet, daß man der Serumprobe 5 bis 10% Vol./Polyglykolmethyläther oder Polyvinylpyrrolidon zusetzt.

6. Verfahren nach einem der vorhergehenden Ansprüche, dadurch gekennzeichnet, daß man der Serumprobe einen Komplexbildner für mehrwertige Kationen zusetzt.

7. Verfahren nach Anspruch 6, dadurch gekennzeichnet, daß man als Komplexbildner ein Polyaminacetat verwendet.

8. Verfahren nach einem der vorhergehenden Ansprüche, dadurch gekennzeichnet, daß man die LDL-Menge bestimmt, indem man den Cholesteringehalt entweder in der Fällung oder im Serum und im Überstand mißt.

9. Verwendung von Polyvinylsulfat mit einwertigem Kation für die Bestimmung der $\beta$-Lipoproteinfraktion (LDL) in Körperflüssigkeiten, insbesondere im Serum.

10. Reagenz für die Bestimmung der $\beta$-Lipoproteinfraktion (LDL) in Körperflüssigkeiten, insbesondere im Serum, dadurch gekennzeichnet, daß es Polyvinylsufat mit einwertigem Kation und zusätzlich einen Polyglycolmethyläther oder/und Polyvinylpyrrolidon enthält.

11. Reagenz nach Anspruch 10, dadurch gekennzeichnet, daß es zusätzlich einen Komplexbildner für mehrwertige Kationen enthält.

12. Reagenz nach Anspruch 11, dadurch gekennzeichnet, daß es 0,1 bis 0,01 Gew.-% Polyvinylsulfat, 5 bis 10 Vol.-% Polyglycolmethyläther oder/und Polyvinylpyrrolidon sowie Komplexbildner für eine Konzentration von 0,01 bis 0,001 Mol, bezogen auf Serumvolumen, enthält.


## Claims

1. Process for the determination of the $\beta$-lipoprotein fraction (LDL) in body fluids, especially in serum, characterised in that one selectively precipitates the LDL fraction from the sample by addition of a polyvinyl sulphate with monovalent cation and determines the LDL in the usual manner.

2. Process according to claim 1, characterised in that one additionally adds a polyglycol methyl ether or polyvinylpyrrolidone to the serum sample.

3. Process according to claim 1 or 2, characterised in that one uses a water-soluble polyvinyl sulphate in which at least 70% of the fundamental vinyl alcohol units carry a sulphate group.

4.Process according to one of the preceding claims, characterised in that one adds 0.1 to 0.10% wt./vol. of polyvinyl sulphate to the sample.

5. Process according to one of claims 2 to 4, characterised in that one adds 5 to 10% vol. of polyglycol methyl ether or polyvinylpyrrolidone to the serum sample.

6. Process according to one of the preceding claims, characterised in that one adds a complex former for polyvalent cations to the serum sample.

7. Process according to claim 6, characterised in that one uses a polyaminoacetate as complex former.

8. Process according to one of the preceding claims, characterised in that one measures the amount of LDL in that one measures the cholesterol content either in the precipitate or in the serum and in the supernatant.

9. Use of polyvinyl sulphate with monovalent cation for the determination of the $\beta$-lipoprotein fraction (LDL) in body fluids, especially in serum.

10. Reagent for the determination of the $\beta$-lipoprotein fraction (LDL) in body fluids, especially in serum, characterised in that it contains polyvinyl sulphate with monovalent cation and additionally a polyglycol methyl ether and/or a polyvinylpyrrolidone.

11. Reagent according to claim 10, characterised in that it additionally contains a complex former for polyvalent cations.

12. Reagent according to claim 11, characterised in that in contains 0.1 to 0.01 wt.% of polyvinyl sulphate, 5 to 10 vol.% of polyglycol methyl ether and/or polyvinylpyrrolidone, as well as a complex former for a concentration of 0.01 to 0.001 mole, referred to the serum volume.


## Revendications

1. Procédé de dosage de la fraction $\beta$-lipoprotéique (LDL) dans les liquides physiologiques, en particulier dans le sérum, caractérisé en ce qu'on précipite sélectivement la fraction LDL à partir de l'échantillon par addition d'un sulfate de polyvinyle à cation monovalent et on dose la LDL de manière habituelle.

2. Procédé suivant la revendication 1, caractérisé en ce qu'on ajoute en outre à l'échantillon de sérum un éther méthylique de polyglycol ou de la polyvinylpyrrolidone.

3. procédé suivant la revendication 1 ou 2, caractérisé en ce qu'on utilise un sulfate de polyvinyle soluble dans l'eau, dans lequel au moins 70% des motifs alcool vinylique qui sont à la base de celui-ci portent un groupe sulfate.

4. Procédé suivant l'une des revendications précédentes, caractérisé en ce qu'on ajoute à l'échantillon de sérum 0,1 à 0,01 en poids/vol. de sulfate de polyvinyle.

5. Procédé suivant l'une des revendications 2 à 4, caractérisé en ce qu'on ajoute à l'échantillon de sérum 5 à 10% en vol. d'éther méthylique de polyglycol ou de polyvinylpyrrolidone.

6. Procédé suivant l'une des revendications précédentes, caractérisé en ce qu'on ajoute à l'échantillon de sérum un complexant pour les cations polyvalents.

7. Procédé suivant la revendication 6, caractérisé en ce qu'on utilise comme complexant un acétate de polyamine.

8. Procédé suivant l'une des revendications précédentes, caractérisé en ce qu'on détermine la quantité de LDL en mesurant la teneur en cholestérol soit dans le précipité, soit dans le sérum et dans le liquide surnageant.

9. Utilisation de sulfate de polyvinyle à cation monovalent pour le dosage de la fraction $\beta$-lipoprotéique (LDL) dans les liquides physiologiques, en particulier dans le sérum.

10. Réactif pour le dosage de la fraction $\beta$-lipoprotéique (LDL) dans les liquides physiologiques, en particulier dans le sérum, caractérisé en ce qu'il contient du sulfate de polyvinyle à cation monovalent et en outre un éther méthylique de polyglycol ou/et de la polyvinylpyrrolidone.

11. Réactif suivant la revendication 10, caractérisé en ce qu'il contient en outre un complexant pour les cations polyvalents.

12. Réactif suivant la revendication 11, caractérisé en ce qu'il contient 0,1 à 0,01% en poids de sulfate de polyvinyle, 5 à 10% en volume d'éther méthylique de polyglycol ou/et de la polyvinylpyrrolidone ainsi qu'un complexant pour une concentration de 0,01 à 0,001 mole, par rapport au volume de sérum.